# EUROPEAN PATENT APPLICATION

(11) **EP 3 564 964 A1**
(43) Date of publication of application: **06.11.2019**
(21) Application number: 18170929.6
(22) Date of filing: 04.05.2018
(51) Int. Cl.: G16H 50/70, G16H 50/20

(54) **METHOD FOR UTILISING NATURAL LANGUAGE PROCESSING TECHNOLOGY IN DECISION-MAKING SUPPORT OF ABNORMAL STATE OF OBJECT**

(71) Applicant: Avaintec Oy, 00180 Helsinki (FI)
(72) Inventor: KUOSMANEN, Pekka, 00100 Helsinki (FI)
(74) Representative: Salomäki, Juha Kari Ensio

(57) **Abstract**

The object of the invention is a method for utilizing natural language processing technology in decision-making support of abnormal state of object. The method comprises the following phases in a way that the output of the previous phase is the input of the next phase: natural language processing (NLP); word2vec model; data preprocessing; word embedding; building LSTM neural network model.

## Description

### FIELD OF THE INVENTION

The object of the invention is a method as defined in the preamble of claim 1 for utilizing natural language processing technology in decision-making support of abnormal state of object. The present invention demonstrates a classification model based on word2vec and long-short term memory, later also shortly "LSTM", to carry on the effective and accurate analysis of abnormal state of object. One embodiment of the object is a human body where for example the method can be used for effective triage analysis of acute abdomen diseases. Invention also presents a computer-based arrangement for creating decision-making support analysis of abnormal state of object.

### BACKGROUND OF THE INVENTION

Acute abdomen diseases are a group of common emergency diseases caused by abdominal pain, containing both simple diseases which has less complications and good prognosis such as appendicitis, and more complex disease which can be life-threatening such as severe acute pancreatitis, perforation of the digestive tract, intestinal ischemic disease, gastrointestinal hemorrhage.

It is reported by U.S., that acute abdomen disease is the third place in adult emergency treatment and it is one of the most common diseases in emergency center, up to 1/4 to 1/5 in emergency diseases, according to some hospitals reports. While there are not many systematic epidemiological investigations of acute abdominal disease in China.

In large hospitals, due to the wide use of unrestricted medical treatment methods, the emergency department is usually very busy, especially during the night after the general outpatient clinic closing. Moreover, emergency doctors have a heavy workload due to the deficiency of medical worker for a long time. In many large hospitals, the number of patients is often more than 100 during the one shift of an emergency doctor, thus, the time of diagnosis for every patient is very limited.

According to the statistics of one emergency department, the average waiting time of every patient was 39 minutes in 2017, and the time of diagnosis for every patient was 12 minutes. The common symptom of acute abdomen disease is abdominal pain. However, acute abdomen diseases contain various kinds of diseases and the symptoms are very different. Thus, under the circumstance of limited diagnosing time and auxiliary examination results, giving an accurate diagnosis, especially to select patients who are in bad health conditions and to provide professional treatment is a very important issue to be solved. This problem can be solved to a large extent to avoid the patient risk caused by delayed diagnosis and misdiagnosis, thus reducing the medial error rate and medical malpractice.

However, it takes a long time for junior doctors to reach the same level of perception which senior physicians have. And what is more, under the high pressure of noisy environment and heavy workload, the ability of integrating information and diagnosing will decline. The rapid and accurate intelligent triage for acute abdomen diseases is a process of classification to predict diagnosis, based on patient's description of symptoms, like chief complaint and history of current illness and few amount of auxiliary examination results.

Medical transcripts and records are a prevalent source of information for analyzing and understanding the state of patients. Medical transcripts are commonly stored as a natural language form. The terminology used in the medical transcripts varies from patient-to-patient due to difference in medical practice. The variation and use of medical terminology requires a trained or skilled medical professional to understand the medical concept relayed by a given transcript. The unstructured nature of the text and the various ways used to refer to the same medical condition make automated analysis challenging. One approach is phrase spotting, such as searching for specific key terms in the medical transcript. The existence of a word or words is used to show the existence of the state of the patient or the existence of the words with other relevant information may be used to infer a state of the patient. Rules are used to determine the contribution of any identified word to the overall inference. Certain conditions may be only implied through a reference to related symptoms or diseases and never mentioned explicitly. The mere presence or absence of certain phrases or words immediately associated to the condition may not be enough to infer the condition of patients.

There are four major challenges for natural language processing: 1) uncertainty is widespread: covering from lexical, syntactic, semantic, pragmatic to phonetic; 2) unpredictability of unknown language phenomena: new vocabulary, terms, semantics and syntax are emerging one after another; 3) always facing data insufficiency: a limited set of language always contains a growing number of language phenomena; 4) the complexity of language knowledge expression: the ambiguity and complexity of semantic knowledge cannot be described in a simple mathematical model, cultural connotations are difficult to describe in a conventional way, semantic calculation requires a large parameter nonlinear calculation.

It can be seen that the natural language processing based on semantic calculation and analysis is expected to get the accurate service approach to human semantic processing mode from computer. The only way to achieve the goal is to understand the mechanism of human brain's understanding of language, so as to realize language cognition form and processing.

In view of the lack of effective solutions to lexical syntactic bottlenecks, it is not possible to establish a knowledge base that meets the needs of artificial intelligence. Natural language processing can only take a different approach, which is to construct a large-scale semantic knowledge base for real corpus. Therefore, statistical methods become the mainstream means of natural language processing. With the help of modern statistics and powerful teaching tools, a complex statistical model can be constructed, and the complex linguistic knowledge (such as syntactic knowledge, etc.) can be integrated into it, and the solution of remote factors can be realized effectively. For the statistical model to achieve greater success, it depends on the breakthrough of linguistic theory and support of the vast language resources that serve natural language process.

The development trend of natural language processing research. In recent years, natural language processing is in a rapid development stage. Various word lists, semantics, grammar dictionaries, corpus and other data resources are increasingly abundant, the emergency of new theories, new methods and new models has promoted the prosperity of natural language processing research. With the popularization of internet technology and the trend of world economic and social integration, there is an urgent need for natural language processing technology, which provides a strong market power for the research and development of natural language processing.

The application of natural language processing to medical texts, especially Chinese medical text processing, is not much. The industry has already had some preliminary work in these areas.

### SUMMARY OF THE INVENTION

The aim of the present invention is to eliminate the aforementioned drawbacks and to provide an inexpensive, functionally reliable and as far as possible discreet method for decision-making support of abnormal state of object. The inventive method is based on a study made by the inventors and later in this application text there are references to this study.

The present invention relates mainly to an arrangement and a method utilizing natural language processing technology in decision-making support of abnormal state of object. One embodiment of the invention is medical decision-making support of acute abdomen diseases. The method mainly uses some professional medical literature and firsthand cases literature written by doctors to form a corpus vectorizing words and then use LSTM model to classify input data. The research shows that the performance of word2vec model constructed with a small amount of professional area texts is better than the general model consisting of a large number of Chinese texts on the classification of medical record.

With the development of deep learning techniques, a large number of models have been developed for the classification of complex in various situations. Among these classification, LSTM model is the preferred method for text comprehension thanks to its characteristics which it can be normalized to vectors of unequal length and also understand the context relationship between vectors to some extent. Our research shows that for small samples and a small amount of learning data, simple LSTM model has been able to obtain better analysis results. However, when the number of learning samples is large (such as Baidu AIP), the recognition effect of the model obtained by using embedding technology is rather poor. This suggests that we should use more targeted data sets to train for specific clinical problems.

Acute abdomen diseases are a series of complicated clinical diseases with different pathogenesis, which can be divided into multiple permutations of anatomical positions and multiple pathogenesis. How to use limited data to describe the anatomical position and pathophysiological process of these diseases has always been a difficult problem. One of the innovations of our study was to use an 8 by 6 matrix to represent the specific clinical classification of diagnosis. This kind of classification can be realized by 8+6=14 binary classifiers. The method of using multi-classifiers to predict complex outcomes is an innovative point in our study. We find that there is a good mapping between vector matrix formed by LSTM model and classifier matrix. For example, in the vector matrix, the distance from the liver to the stomach is closer to the liver to the gallbladder. The classifier result indicates that the gallbladder and stomach are prone to perforate, while the liver is not. This suggests that our LSTM technology is likely to be able to perceive some certain relationships within the concept of classification of disease in certain organs.

At the same time, diagnosing acute abdomen diseases is a very complicated process of information processing. The text alone cannot obtain all clinical information of the patients. In the context of acute abdomen diseases, the process of information collecting is often accompanied by the clinical decision-making process for doctors. In this case, the high clinical diagnosis rate obtained from classification of patients' information collected by doctors via natural language processing does not mean what we can work through similar technology to replace the doctors' work. The above research only means that we can excavate important information from text analysis that can carry out clinical clues. The purpose is to establish an intelligent rapid diagnosis system of acute abdominal pain diseases based on natural language processing and deep learning technology in order to promptly and accurately evaluate the patient's condition, and shorten the treatment process of emergency patient, which is crucial for the treatment of patients at the maximin extent by saving time, meanwhile, medical disputes can be avoided and medical resources are rationally allocated.

The invention also comprises a computer based arrangement for creating decision-making support analysis of abnormal state of object, which arrangement comprises for example a database file containing corpus to be treated, computer memory for storing the classifications and results, a processor for carrying out the instructions of a computer program written in accordance with the present invention, and means for outputting the results of the classification to a human operator or to a storage file (e.g. a database file) .

### LIST OF FIGURES

In the following, the invention will be described in greater detail by the aid of an example of its embodiment with reference to the attached drawings, wherein
- Fig. 1: presents one form of Chinese word segmentation, and
- Fig. 2: presents another form of Chinese word segmentation.

### DETAILED DESCRIPTION OF THE INVENTION

This example is focusing for achieving the decision-making support diagnosis of acute abdomen diseases from Chinese medical transcriptions and medical records. Chinese has no spaces among every word. In order to extract meaningful words for build model, Chinese sentences should be segmented in medical records first of all by natural language processing.

In this example there are total 922 medical records data of inpatients with acute abdominal pain in Chinese Sichuan People Hospital in 2015-2017, including patients' name, age, gender, chief complaint, present and past history of illness and diagnosis.
Inclusion criteria: 1) acute abdominal pain; 2) adult patients (age>18);
Exclusion criteria: 1) age<18; 2) chronic abdominal pain

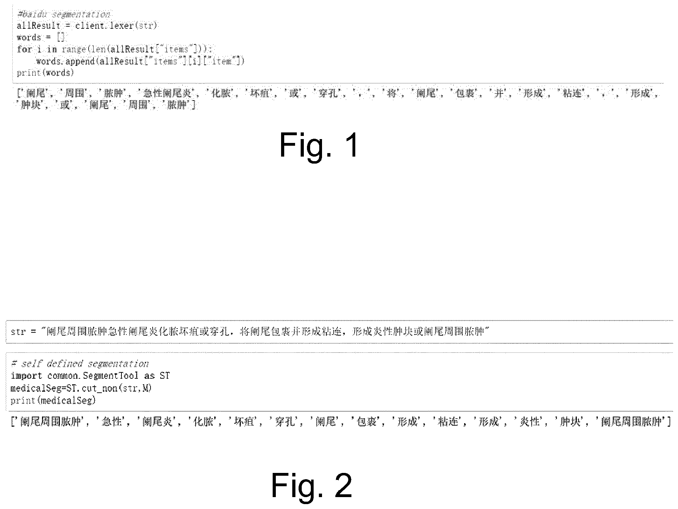

The invention is based on following five phases:
1) Natural language processing (NLP): according to some medical literature or teaching materials, the medical terms were extracted and added to the Chinese word segmentation tool to construct the custom medical Chinese word segmentation tool. The medical classification, namely, the Chinese word segmentation tool is constructed by using the customized corpus.
   Using the Baidu Chinese word segmentation tool, the sentence "periappendicural absecess, acute appendicitis, pyogenic gangrene or perforation, the appendix is wrapped, and adhesion formed, forming an inflammatory mass or an abscess around the appendix" can be divided into the form presented in figure 1. Figure 2 adopts the customized medical Chinese word segmentation tool, which can be divided into the forms presented in figure 2. It can be seen, that the medical Chinese word segmentation tool is slightly better than the Chinese word segmentation tool established by Baidu's nonspecific corpus.
2) Word2vector model: using customized segmentation tools to remove some stop words and irrelevant words in the corpus containing medical literature and medical text books. Then, using corpus build a word2vector model after word segmentation, which could represent words by vectors, and eventually project as a multiple-dimensional matrix.
   The text entities of electronic medical records are extracted by using the special word segmentation tool, and the distance between a disease and symptoms in corpus matrix is calculated, which connect those two entities. Usually there is only one relationship between a disease and the symptoms, which is that a disease causes the symptoms. In other words, the relationship is that a diagnosis of a disease because of the occurrence of symptoms.
   For example, cholecystitis. Reminder: the right kidney increases with its internal density to reduce disease, "cholecystitis "causes symptom group {"the right kidney increases", "the density decreases"}; or "acute appendicitis" in the upper abdominal pain, dull pain, with nausea, vomiting, diarrhea and other symptoms, that is "acute appendicitis" causes the symptom group{"upper abdominal pain", "nausea", "vomiting", "diarrhea"}. We put diagnosis and symptoms into the corpus model to calculate the distance between them, so as to obtain the most likely diagnosis of different symptoms.
3) Data preprocessing: in the early stage, the main complaint, present medical history and past medical history in the 922 data were processed by using the customized Chinese word segmentation tool for Chinese word segmentation.
4) Word embedding: the data of medical records after segmentation is vectorized by word2vector model and output a matrix. Afterwards, a 922*n matrix regards as training data.
5) Utilizing supervised machine learning techniques to obtain a classification model based on the available dataset.

The general problem described in this invention requires establishing a correct assignment between medical records and corresponding medical diagnosis, i.e. the disease of the person. In general, artificial intelligence and machine learning terminology, this is a supervised learning task: based on a set of datapoints (e.g. medical records represented as vectors as described in the previous steps), one needs to derive a model that can be used to identify the correct class that a datapoint belongs to (e.g. disease corresponding to the record). This can be performed by utilizing different well-established classification methods, such as logistic regression, support vector machines, or recurrent neural network models, such as Long Short Term Memory networks by building LSTM neural network model to classify input data. Based on the assessment of the performance of various models, an optimal one can be chosen using traditional evaluation measures, such as accuracy, recall or precision.

It is obvious to the person skilled in the art that different embodiments and applications of the invention are not limited to the example described above, but that they may be varied within the scope of the claims presented below. Thus, for example, the method can be used for achieving the decision-making support analysis of computer related problems in help-desk.

## Claims

1. A method for utilizing natural language processing technology in decision-making support of abnormal state of object, **characterized in that** the method comprises the following phases in a way that the output of the previous phase is the input of the next phase:
- natural language processing (NLP),
- word2vec model,
- data preprocessing,
- word embedding,
- building LSTM neural network model.

2. Method according to claim 1, **characterized in that** the corpus vectorising words is generated by word2vec model, that is constructed with a small amount of professional area texts.

3. Method according to claim 1 or 2, **characterized in that** an 8 by 6 matrix to is used to represent the specific classification of analysis.

4. Method according to any of the claims above, **characterized in that** multi-classifiers are used to predict complex outcomes.

5. Method according to any of the claims above, **characterized in that** 8+6=14 binary classifiers are used to predict complex outcomes.

6. Method according to any of the claims above, **characterized in that** medical transcripts and records are used as a corpus.

7. **Use** of a method according to any of claims 1-6 for achieving the decision-making support diagnosis of acute abdomen diseases.

8. Use of a method according to any of claims 1-5 for achieving the decision-making support analysis of computer related problems in help-desk.

9. Use of a method according to any of claims 1-8, **characterized in that** Chinese language text is used as a corpus.

10. Computer-based **arrangement** according to any of methods and/or uses above for achieving the decision-making support analysis of abnormal state of object, which arrangement comprises a database file containing corpus to be treated, computer memory for storing the classifications and results, a processor for carrying out the instructions of a computer program written in accordance with the present invention, and means for outputting the results of the classification to a human operator or to a storage file.
